# EUROPÄISCHE PATENTSCHRIFT

(11) **EP 2 530 074 B1**
(45) Veröffentlichungstag und Bekanntmachung des Hinweises auf die Patenterteilung: **23.10.2013**
(21) Anmeldenummer: 11168080.7
(22) Anmeldetag: 30.05.2011
(51) Int. Cl.: C07D 201/02, C08G 69/16

(54) **Neue Zusammensetzung zur Herstellung von Gusspolyamiden**
New compounds for producing poured polyamides
Nouvelle composition pour la fabrication de polyamides de fonte

(43) Veröffentlichungstag der Anmeldung: 05.12.2012
(73) Patentinhaber: Rhein Chemie Rheinau GmbH, 68219 Mannheim (DE)
(72) Erfinder: Laufer, Wilhelm, Dr., 67158 Ellerstadt (DE); Bechem, Benjamin, Dr., 68165 Mannheim (DE); Palzer, Andre, 68782 Brühl (DE)
(74) Vertreter: Siegers, Britta

(56) Entgegenhaltungen:
- EP-A1- 0 459 199
- US-A- 5 264 479

## Beschreibung

Die vorliegenden Erfindung betrifft neue Zusammensetzungen für die Herstellung von Gusspolyamiden.

Gusspolyamide sind besonders hochmolekulare Polyamide. Bei der Herstellung von Gusspolyamiden wird ein Lactam zusammen mit mindestens einem Katalysator und mindestens einem Aktivator in eine Form gegossen und dann in dieser Form anionisch polymerisiert. Die in der Form vorliegenden Ausgangsverbindungen polymerisieren dabei im Allgemeinen unter der Einwirkung von Wärme. Dabei entsteht ein homogener Werkstoff, welcher extrudierte Polyamide im Hinblick auf die Kristallinität übertrifft.

Gusspolyamide sind als thermoplastische Kunststoffe für die Fertigung komplexer Bauteile geeignet. Sie müssen im Gegensatz zu vielen anderen Thermoplasten nicht aufgeschmolzen werden, sondern entstehen durch eine drucklose anionische Polymerisation von einem Lactam in einer Form bei 120 bis 160°C bereits in wenigen Minuten. Dabei können alle bekannten Gießverfahren, wie Standguss, Rotations- und Schleuderguss, angewendet werden. Als Endprodukt erhält man jeweils Formteile aus einem hochmolekularen, kristallinen Polyamid, das sich durch ein niedriges Gewicht, eine hohe mechanische Belastbarkeit, sehr gute Gleiteigenschaften und eine hervorragende Chemikalienbeständigkeit auszeichnet und das - da die Formen nicht unter Druck gefüllt werden - nur geringe innere Spannungen aufweist. Gusspolyamide lassen sich Sägen, Bohren, Fräsen, Schleifen, Verschweißen und Bedrucken oder Lackieren; neben komplexen Hohlformen werden aus diesem Polymer beispielsweise auch Rollen für Personenaufzüge und Halbzeuge, wie zum Beispiel Rohre, Stäbe und Platten für den Maschinenbau und die Automobilindustrie, gefertigt.

Die Herstellung von Gusspolyamidteilen, ausgehend von niedrig viskosen Lactamschmelzen und einem Katalysator sowie einem Aktivator durch die sogenannte aktivierte anionische Polymerisation, ist an sich bekannt. Zu diesem Zweck werden üblicherweise zwei Mischungen aus Katalysator und Lactam bzw. Aktivator und Lactam in Form einer flüssigen Schmelze frisch vor der Polymerisation getrennt voneinander hergestellt, unmittelbar miteinander vermischt und anschließend in der Gussform polymerisiert. Dies soll sicherstellen, dass es im Vorfeld zu keinen unerwünschten Reaktion kommt.

Die im Stand der Technik bekannten Zusammensetzungen haben die Nachteile, dass diese entweder feste Aktivatoren mit geringer Aktivität oder lösemittelhaltige flüssige Polyisocyanate beinhalten.

Eine Aufgabe der vorliegenden Erfindung besteht somit darin, Zusammensetzungen bereitzustellen, die die Nachteile des Standes der Technik nicht aufweisen.

Überraschenderweise wurde nun gefunden, dass Zusammensetzungen, enthaltend
a) mindestens ein Lactam und
b) mindestens einen Aktivator für die anionische Polymerisation von Lactamen der Formel (I) 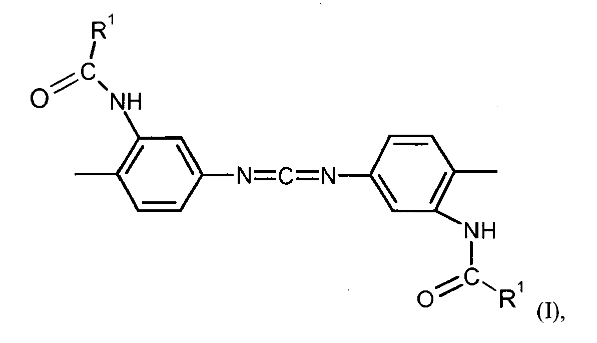 mit R¹ = Lactam, bevorzugt Caprolactam, die Nachteile des Standes der Technik nicht aufweisen.

Gegenstand der vorliegenden Erfindung sind daher Zusammensetzungen, enthaltend
a) mindestens ein Lactam und
b) mindestens einen Aktivator für die anionische Polymerisation von Lactamen, wobei es sich bei dem Aktivator um eine Verbindung der Formel (I) 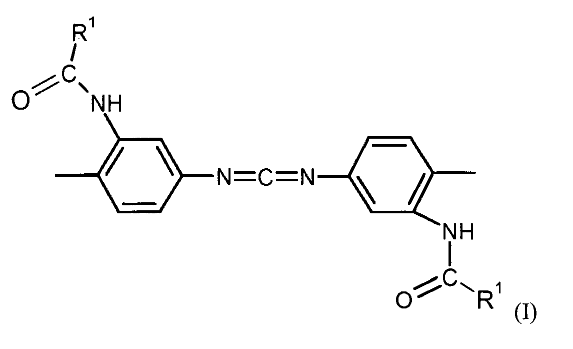
mit R¹ = Lactam, bevorzugt Caprolactam, handelt.

In einer weiteren bevorzugten Ausführungsform der Erfindung handelt es sich bei dem Aktivator um eine Verbindung der Formel (II)

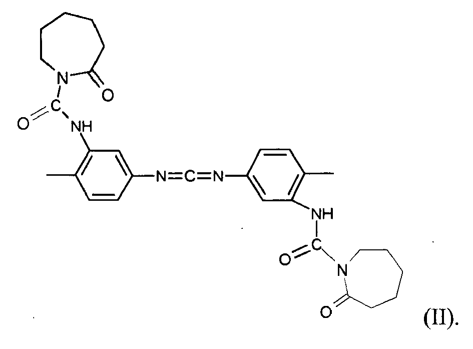

Bei den Aktivatoren handelt es sich vorzugsweise um Verbindungen, die durch Umsetzung von N,N'-bis(3-isocyanato-4-methylphenyl)carbodiimid mit Caprolactam nach dem, dem Fachmann geläufigen Verfahren erhältlich sind.

Vorzugsweise erfolgt die Herstellung in Gegenwart eines Lösemittels wie z. B. Benzin, oder Alkylbenzolen, wie z.B. Toluol oder Xylol.

Als Lactam im Sinne der Erfindung können Verbindungen der allgemeinen Formel (III) 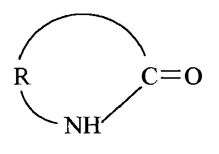 eingesetzt werden, wobei R eine Alkylengruppe mit 3 bis 13 Kohlenstoffatomen darstellt. Bevorzugt handelt es sich dabei um Capro-Lactam und/oder Laurin-Lactam. Diese sind kommerziell erhältlich z.B. bei der Firma Lanxess Deutschland GmbH.

Die vorgenannten Verbindungen sind handelsüblich und sind beispielsweise bei der Firma Rhein Chemie Rheinau GmbH oder bei der Bayer MaterialScience AG erhältlich.

In einer weiteren Ausführungsform der Erfindung enthält die Zusammensetzung zusätzlich mindestens einen Katalysator, ausgewählt aus der Gruppe Lactam-Magnesium-Halogenid, Alkali-Alumo-Dilactamat, Alkali- und/oder Erdalkalilactamat. Besonders bevorzugt dabei ist Natriumcaprolactamat.

Die vorgenannten Katalysatoren sind handelsüblich und sind beispielsweise bei der Firma Rhein Chemie Rheinau GmbH oder bei der Firma KatChem spol.s.r.o. erhältlich.

In einer weiteren Ausführungsform der Erfindung weist die Zusammensetzung folgende Anteile an a) bis c) auf,
a) 94 - 99,9 % mindestens eines Lactams,
b) 0,1 - 5% , bevorzugt 0,2 - 2%, besonders bevorzugt 0,2 bis 0,8%, mindestens eines Aktivators und
c) 0 - 5% mindestens eines Katalysators,
wobei die Summe der Bestandteile 100% beträgt.

Bei den Prozentangaben handelt es sich um Gew.%.

In einer weiteren Ausführungsform der Erfindung enthält die erfindungsgemäße Zusammensetzung noch weitere Additive, wie Schlagzähmodifikatoren, wie z.B. Polyetheramin-Copolymere, Glasfasern, Endlosglassfasern, Kohlefasern, Aramidfasern und/oder Verarbeitungshilfsmittel, wie z.B. hochmolekulare Polyole, Verdicker, wie z.B. Aerosile, UV- und Thermostabilisatoren, Leitfähigkeitsverbesserer, wie z.B. Russe und Graphite, ionische Flüssigkeiten, Markierungsstoffe und/oder Farben.

Gegenstand der vorliegenden Erfindungen ist zudem ein Verfahren zur Herstellung von Gußpolyamiden, die Bestandteile a) und b) und gegebenenfalls c) der erfindungsgemäßen Zusammensetzung als Schmelze bei Temperaturen von 80 - 160°C zur Polymerisation gebracht werden.

In den Fällen, in denen die erfindungsgemäße Zusammensetzung noch weitere Additive enthält können diese vor und/oder während der Polymerisation zugegeben werden.

Die Menge an Additiven wird vom Einsatzzweck bestimmt und kann daher beliebig variiert werden.

Die Polymerisation erfolgt nach den, dem Fachmann geläufigen Verfahren, wie z.B. beschrieben in Kunststoffhandbuch, Bd.3/4, Technische Thermoplaste, Hanser Fachbuch, Seiten 413 - 430. Dabei wird die Mischung vorzugsweise gerührt. Einsetzbar hierfür sind Mischaggregate, wie z.B. Rührkesseln.

Ein weiterer Gegenstand der vorliegenden Erfindung ist die Verwendung erfindungsgemäßen Zusammensetzung zur Herstellung von Gusspolyamiden.

Die vorgenannten erfindungsgemäßen Zusammensetzungen werden vorzugsweise als Ersatz für Metall, z.B. in der Automobilindustrie, bei der Produktion von elektrotechnischen Teilen und für elektronische Zwecke, für die Herstellung von Platten, Stäben, Rohren, Seilscheiben, Seilrollen, Zahnrädern und Lagern und/oder für die Behälterfertigung eingesetzt.

Der Rahmen der Erfindung erfasst alle oben stehenden und im Folgenden aufgeführten allgemeinen oder in Vorzugsbereichen genannten Restedefinitionen, Indizes, Parameter und Erläuterungen untereinander, also auch zwischen den jeweiligen Bereichen und Vorzugsbereichen in beliebiger Kombination.

Die nachfolgenden Beispiele dienen der Erläuterung der Erfindung, ohne dabei limitierend zu wirken.

### Ausführungsbeispiele:

### Reagenzien:

Caprolactam trocken (EP > 69 °C) von der Firma Lanxess Deutschland GmbH.

Als Katalysator wird eingesetzt: Addonyl® Kat NL von der Rhein Chemie Rheinau GmbH, ca. 18 % Natriumcaprolactamat in Caprolactam.

Als Aktivatoren werden eingesetzt:
1) Caprolactam blockiertes N,N'-bis(3-isocyanato-4-methylphenyl)carbodiimid gemäß Formel (II) (erfindungsgemäß) siehe Bsp. 1,
2) Addonyl® 8108: aliphatische Polyisocyanatlösung, kommerziell erhältlich bei der Rhein Chemie Rheinau GmbH, siehe Bsp. 2,
3) Brüggolen® C20 P: Caprolactam blockiertes Hexamethylendiisocyanat in Caprolactam, kommerziell erhältlich von der Firma Brüggemann GmbH, siehe Bsp. 3,
4) N,N'-bis(3-isocyanato-4-methylphenyl)carbodiimid: Monomeres Carbodiimid auf Basis 2,4-Toluylendiisocyanat, siehe Bsp. 4,
5) Stabaxol® P: aromatisches Polycarbodiimid auf Basis von 1,3,5-Triisopropyl-2,4-diisocyanatobenzol, siehe Bsp. 5,
6) Addolink® TT: dimeres TDI-Urethdion, siehe Bsp. 6.

Geräte:
Die verwendete Apparatur zur Schmelzaufbereitung bestand aus:
   - 2 Dreihalskolben (500 ml) im Ölbad beheizt
   - 2 KPG-Rührer mit Hülse
   - 2 Gaskappen je 1 mit und 1 ohne Hahn
   - 1 Vakuumpumpe mit Kühlfalle und Manometer.

Die verwendete Apparatur zur Temperaturmessung bestand aus:
- Temperaturmessgerät, z.B. Testo 175-T3 mit IR-Serial Interface
- Thermodraht zum Verbleib in der ausgehärteten Probe
- Becherglas 600 ml (hohe Form) und einer
- Beheizung für das Becherglas (Metallblock, Ölbad).

### Durchführung und Messung:

Kolben A wurde mit 196,8 g Caprolactam und Aktivator, Kolben B mit 192 g Caprolactam und 8 g Katalysator NL-Neu beschickt.

Die Schmelzen aus den Kolben A und B wurden bei 122 °C (± 2 °C) in einem Ölbad unter Vakuum (<15 mbar) 20 Minuten aufbereitet.

Nach Belüften mit Stickstoff wurden Komponenten aus Kolben A und Kolben B in einen Dreihalskolben vereinigt, kurz durchgerührt und in das 600 ml Becherglas überführt.

Die Formtemperatur (Becherglas) betrug 160° C. Die Polymerisationszeit betrugt in der Regel 10 - 20 Minuten.

Tabelle 1 zeigt die Ergebnisse.

| **Aktivator** | **Beispiel 1** | **Beispiel 2** | **Beispiel 3** | **Beispiel 4** | **Beispiel 5** | **Beispiel 6** |
|---|---|---|---|---|---|---|
| | (erf) | (vgl) | (vgl) | (vgl) | (vgl) | (vgl) |
| **0,8 Gew. %** | Guss-PA* | Guss-PA | Guss-PA | kein Guss-PA | Guss-PA | Guss-PA |
| **0,5 Gew. %** | Guss-PA | Guss-PA | Guss-PA | | kein Guss-PA | kein Guss-PA |
| **0,3 Gew. %** | Guss-PA | Guss-PA | kein Guss-PA | | | |
| **0,2 Gew. %** | Guss-PA | Guss-PA | | | | |
| 0**,1Gew. %** | kein Guss-PA** | kein Guss-PA | | | | |

| | | | | | | |
|---|---|---|---|---|---|---|
| * Herstellung von Gußpolyamidteilen erfolgreich ** keine oder unvollstandige Polymensation | | | | | | |

Die Beispiele zeigen, dass die erfindungsgemäße Zusammensetzung auch bei sehr geringen Aktivator-Konzentrationen die Herstellung von Gusspolyamidteilen ermöglicht. Ähnlich gute Ergebnisse können lediglich bei Verwendung von Polyisocyanatlösungen als Aktivatorkomponente erreicht werden. Diese haben jedoch den Nachteil lösemittelhaltig zu sein.

## Patentansprüche

1. Zusammensetzung, enthaltend
a) mindestens ein Lactam und
b) mindestens einen Aktivator für die anionische Polymerisation von Lactamen, **dadurch gekennzeichnet, dass**
der es sich bei dem Aktivator um eine Verbindung der Formel (I) 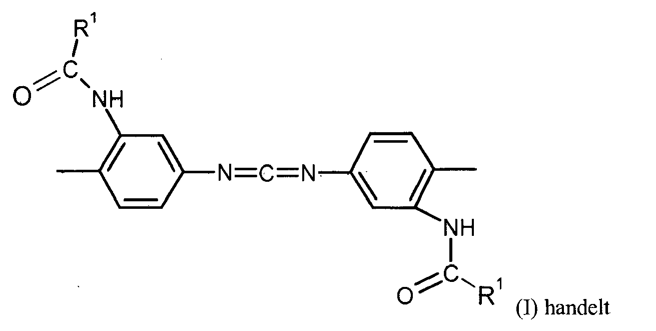 mit R¹ = Lactam, bevorzugt Caprolactam.

2. Zusammensetzung nach Anspruch 1, **dadurch gekennzeichnet, dass** es sich bei dem Aktivator um eine Verbindung der Formel (II)

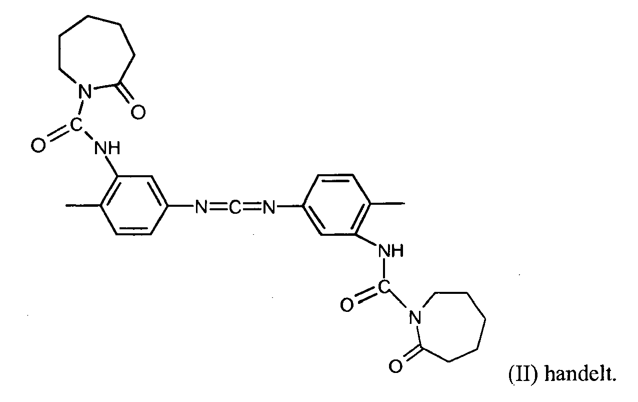

3. Zusammensetzung nach einem oder mehreren der Ansprüche 1 bis 2, **dadurch gekennzeichnet, dass** es sich bei dem Lactam um eine Verbindung der allgemeinen Formel (III) 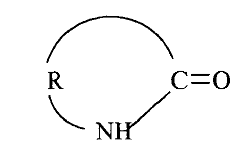 handelt, wobei R eine Alkylengruppe mit 3 bis 13 Kohlenstoffatomen darstellt.

4. Zusammensetzung nach einem oder mehreren der Ansprüche 1 bis 3, **dadurch gekennzeichnet, dass** diese zusätzlich mindestens einen Katalysator ausgewählt aus der Gruppe Lactam- Magnesium- Halogenid, Alkali-Alumo-Dilactamat, Alkali- und/oder Erdalkalilactamat enthält.

5. Zusammensetzung nach einem oder mehreren der Ansprüche 1 bis 4, **dadurch gekennzeichnet, dass** diese folgende Zusammensetzung aufweist:
a) 94 - 99,9 % mindestens eines Lactams,
b) 0,1 - 5% mindestens eines Aktivators und
c) 0 - 5% mindestens eines Katalysators ,
wobei die Summe der Bestandteile a) bis c) 100% beträgt.

6. Verfahren zur Herstellung von Gusspolyamiden nach einem oder mehreren der Ansprüche 1 bis 5, **dadurch gekennzeichnet, dass** die Bestandteile a) und b) und gegebenenfalls c) der Zusammensetzung nach einem oder mehreren der Ansprüche 1 bis 5 als Schmelze bei Temperaturen von 80 - 160°C zur Polymerisation gebracht werden.

7. Verwendung der Zusammensetzung nach einem oder mehreren der Ansprüche 1 bis 5 zur Herstellung von Gusspolyamiden.

## Claims

1. Composition comprising
a) at least one lactam and
b) at least one activator for the anionic polymerization of lactams,
**characterized in that**
the activator involves a compound of the formula (I) 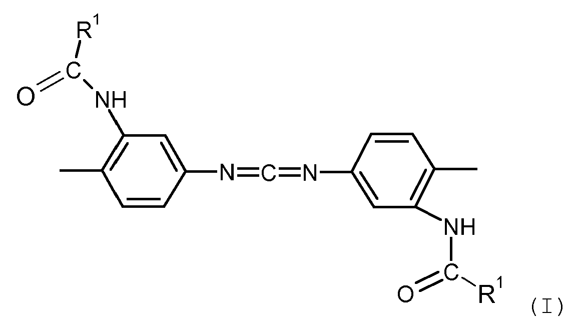 where R¹ = lactam, preferably caprolactam.

2. Composition according to Claim 1, **characterized in that** the activator involves a compound of the formula (II)

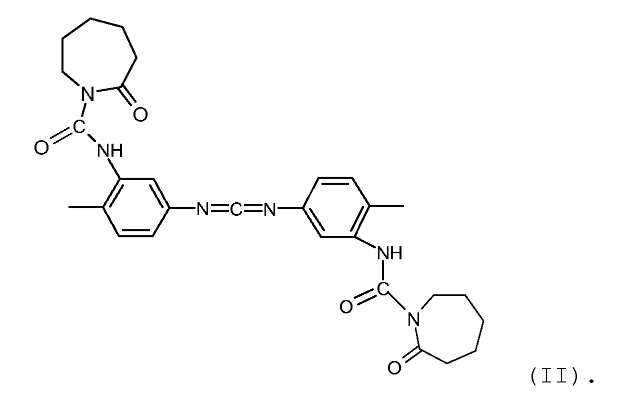

3. Composition according to one or more of Claims 1 to 2, **characterized in that** the lactam involves a compound of the general formula (III) 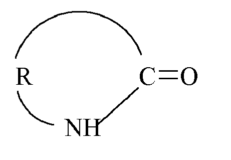 where R is an alkylene group having from 3 to 13 carbon atoms.

4. Composition according to one or more of Claims 1 to 3, **characterized in that** this also comprises at least one catalyst selected from the group of lactam magnesium halide, alkali metal aluminodilactamate, alkali metal lactamate, and/or alkaline earth metal lactamate.

5. Composition according to one or more of Claims 1 to 4, **characterized in that** this is constituted as follows:
a) from 94 to 99.9% of at least one lactam,
b) from 0.1 to 5% of at least one activator, and
c) from 0 to 5% of at least one catalyst,
where the entirety of the constituents a) to c) is 100%.

6. Process for producing cast polyamides according to one or more of Claims 1 to 5, **characterized in that** the constituents a) and b) and optionally c) of the composition according to one or more of Claims 1 to 5 are polymerized at temperatures of from 80 to 160°C in the form of melt.

7. Use of the composition according to one or more of Claims 1 to 5 for producing cast polyamides.

## Revendications

1. Composition contenant
a) au moins un lactame et
b) au moins un activateur pour la polymérisation anionique de lactames,
**caractérisée en ce qu'**il s'agit, pour l'activateur, d'un composé de formule (I) 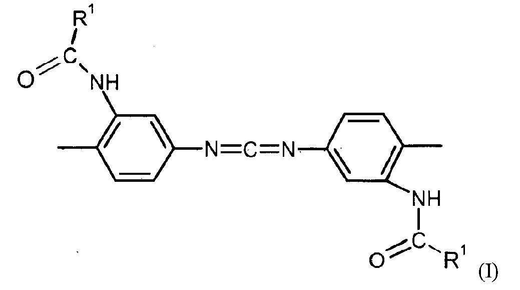 R¹ = lactame, de préférence caprolactame.

2. Composition selon la revendication 1, **caractérisée en ce qu'**il s'agit, pour l'activateur, d'un composé de formule (II)

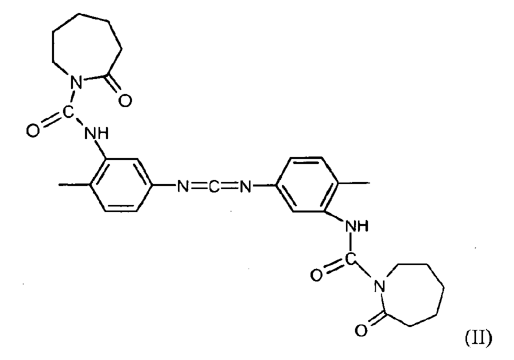

3. Composition selon l'une ou plusieurs des revendications 1 à 2, **caractérisée en ce qu'**il s'agit, pour le lactame, d'un composé de formule générale (III) 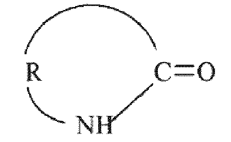 où R représente un groupe alkylène comprenant 3 à 13 atomes de carbone.

4. Composition selon l'une ou plusieurs des revendications 1 à 3, **caractérisée en ce qu'**il contient en outre au moins un catalyseur choisi dans le groupe formé par un halogénure de lactame-magnésium, un alumino-dilactamate de métal alcalin, un lactamate de métal alcalin et/ou de métal alcalino-terreux.

5. Composition selon l'une ou plusieurs des revendications 1 à 4, **caractérisée en ce qu'**elle présente la composition suivante :
a) 94-99,9% d'au moins un lactame,
b) 0,1-5% d'au moins un activateur et
c) 0-5% d'au moins un catalyseur,
la somme des constituants a) à c) valant 100%.

6. Procédé pour la préparation de polyamides à couler selon l'une ou plusieurs des revendications 1 à 5, **caractérisé en ce que** les constituants a) et b) et le cas échéant c) de la composition selon l'une ou plusieurs des revendications 1 à 5 sont amenés à polymériser sous forme de masse fondue à des températures de 80-160°C.

7. Utilisation de la composition selon l'une ou plusieurs des revendications 1 à 5 pour la préparation de polyamides à couler.
